# EUROPEAN PATENT SPECIFICATION

(11) **EP 2 446 908 B1**
(45) Date of publication and mention of the grant of the patent: **15.07.2015**
(21) Application number: 11161646.2
(22) Date of filing: 08.04.2011
(51) Int. Cl.: A61M 1/16, A61M 1/36, A61M 1/34, B01J 20/06, B01J 20/12, B01J 20/18, B01J 20/22, B01J 20/24, B01J 20/26, B01J 20/28, B01J 20/32, B01D 61/14, B01D 61/24, B01D 61/28, B82Y 30/00, B01J 20/20, A61M 1/14

(54) **Electrosorption and decomposition device for the purification of blood and other fluids**
Elektrosorptions- und Zersetzungsvorrichtung zur Reinigung von Blut und anderen Flüssigkeiten
Dispositif d'électrosorption et décomposition pour la purification du sang et d'autres fluides

(30) Priority: 02.11.2010 EP 10189728
(43) Date of publication of application: 02.05.2012
(73) Proprietor: ICinnovation BV, 5688 RX Oirschot (NL)
(72) Inventor: Simonis, Frank, 5688 RX, Oirschot (NL)
(74) Representative: Verhees, Godefridus Josephus Maria

(56) References cited:
- EP-A1- 1 745 808
- GB-A- 1 406 133
- GB-A- 2 000 150
- US-A- 3 827 961

## Description

### FIELD OF THE INVENTION

The present invention is in the field of purification of fluids by electrosorption and electrocatalytic decomposition, in particular but not limited to biofluids such as blood, as of use in artificial kidneys, artificial livers and hemodialysis systems. The invention relates to a stand-alone device for the removal of toxic substances from a biofluid, such as from the blood from a patient, to methods of removing toxic substances from blood using the inventive device in a wearable unit enabling continuous blood purification while being mobile. The invention is also applicable to other fluids such as waste water, process chemicals and other biofluids such as removing substances from urine, dialysate fluids, milk, biochemical analysis and processing fluids. In a reverse mode the invented system can be used to release ingredients in a controlled manner.

### BACKGROUND OF THE INVENTION

Hemodialysis (HD) and peritoneal dialyis (PD) are methods of removing toxic substances (impurities or wastes) from the blood when the kidneys are unable to do so sufficiently. Dialysis is most frequently used for patients who have kidney failure, but may also be used to quickly remove drugs or poisons in acute situations. This technique can be life saving in people with acute or chronic kidney failure. Best known is hemodialysis, which works by circulating the blood along special filters outside the body in a dialysis machine. Here, the blood flows across a semi-permeable membrane (the dialyser or filter), on the other side of which flows a dialysis fluid in a counter-current direction to the blood flow. The dialysing membrane allows passage of substances below a certain molecular cutoff. By diffusion the concentration of these substances will end up being the same on both sides of the membrane. The dialysis fluid removes the toxins from the blood and is generally discarded as waste dialysate. The chemical imbalances and impurities of the blood are being brought back in minimal balance and the blood is then returned to the body. The efficacy of hemodialysis is 10 - 15%, which means that 10 - 15% of the toxins are being removed from the blood. Typically, most patients undergo hemodialysis for three sessions every week. Each session lasts normally 3-4 hours. This is very inconvenient, and the physical and social side effects of dialysis to the patients are a great concern.

In order to provide for portable dialysis devices, that will allow patients to engage in normal daily activities, artificial kidneys have been developed. Essentially there are two types of artificial kidneys.

In one form, the principle of the artificial kidney consists of extracting urea and other more toxic middle molecules from blood by dialysis and regeneration of the dialysate by means of an adsorbent, usually activated carbon. In the case of a system based on such a dialysis kidney machine, a key aspect resides in regenerating the dialysis fluid when the latter is to be recycled into the dialyser. Dialysis kidney machines that can be encountered in the prior art include for instance those described in GB 1 406 133, and US 2003/0097086. GB 1 406 133 discloses an artificial kidney of the recycle type having an improved adsorbent comprising activated carbon and alumina. US 2003/0097086 discloses a portable dialysis device comprising dialyzers connected in series that utilize dialysate, and further comprising a plurality of contoured sorbent devices, which are connected in series and are for regenerating the spent dialysate. As adsorption materials for regeneration of the spent dialysate, activated charcoal, urease, zirconium phosphate, hydrous zirconium oxide and/or activated carbon are provided.

In another form, the principle of the artificial kidney may be based on ultrafiltration, or hemofiltration, using appropriate membranes, wherein large molecules including blood cells are retained in the retentate on the filter, and the toxic substances are collected in the (ultra)filtrate. During hemofiltration, a patient's blood is passed through a set of tubing (a filtration circuit) via a machine to a semipermeable membrane (the filter) where waste products and water are removed. Replacement fluid is added and the blood is returned to the patient. In a similar fashion to dialysis, hemofiltration involves the movement of solutes across a semi-permeable membrane. However, the membrane used in hemofiltration is far more porous than that used in hemodialysis, and no dialysate is used-instead a positive hydrostatic pressure drives water and solutes across the filter membrane where they are drained away as filtrate. An isotonic replacement fluid is added to the resultant filtered blood to replace fluid volume and valuable electrolytes. This is then returned to the patient. Thus, in the case of ultrafiltration, a key aspect resides in separating the urea from the other components in the ultrafiltrate such as salts which have also passed through the membrane but which must be reincorporated into the blood in order to maintain the electrolyte composition thereof substantially constant.

A combination of the two systems described above has also been proposed. Shettigar and Reul (Artif. Organs (1982) 6:17-22), for instance, disclose a system for simultaneous filtration of blood using a hemofilter and dialysis against its purified filtrate, wherein the filtrate is purified by a multi-adsorption system consisting of charcoal for removal of urea and a cation exchanger.

Intermediate systems, i.e. systems that perform no ultrafiltration, yet which adsorb toxic substances directly from the blood have also been proposed. US 2004/0147900 discloses a cartridge for treating medical or biological fluid, in particular blood, consisting of a compartmentalized container, wherein each compartment contains adsorbing particles. The adsorption materials proposed are essentially those disclosed in US 2003/0097086 described above, and thus may effectively remove urea from blood.

As noted above, the adsorbent for regenerating the dialysate is usually activated carbon. However other adsorbents have been proposed for the removal of substances from dialysis fluids or ultrafiltrate. US 3,874,907, for instance, discloses microcapsules consisting essentially of a crosslinked polymer containing sulphonic acid groups and coated with a polymer containing quaternary ammonium groups, for use in an artificial kidney. Examples of the sulphonated polymer include sulphonated styrene/divinyl benzene copolymer and examples of the coating polymer include those obtained by polymerization of for instance vinyldimethylamine monomers. Shimizu et al. (Nippon Kagaku Kaishi (1985), (6), 1278-84) described a chemisorbent composition for the removal of urea from dialysis fluid or hemofiltrate for use in an artificial kidney. The chemisorbent is based on dialdehyde starch (DAS)-urease conjugates and 4,4'-diamidinodiphenylmethane (DADPM).

Another approach relates to electrodialysis, a method to fasten the dialysis process by applying an electrical field over the dialysate membrane similar to electrophoresis systems. For instance in WO03020403 a dialysate system is proposed with an electrical voltage over the membrane. The proposed voltage is in the range of 50-150 Volts. It is claimed that the electrical field promotes the diffusion rate and hereby the clearance rate of toxins such as small solutes, phosphate, creatinine, beta2microglobuline and even urea. A major drawback however is the required high voltage resulting in significant heating of the blood. This system therefore requires an additional cooling section making the system bulky and energy consuming.

Another relevant approach is known from purification of water and is called electrosorption. E.g. in US2007/00728431 an apparatus is disclosed for removing inorganic ions such as salt and metals from water by means of carbon electrodes that are activated with a small voltage. This system seems to work well for water and inorganic substances. Nothing has been disclosed so far for removing organic molecules and substances such as toxic small and middle molecules and proteins via electrosorption.

In conclusion, the prior art discloses both dialysing and ultrafiltration devices, wherein various substances may be used as sorbents. Also the use of an external electrical field to boost the dialysate diffusion process has been disclosed.

US 3827961 describes an electrosorption device used for the purification of dialysis fluid.

The problem with the system of the prior art is that however, that they are still too large due to limited sorption capacity of the materials, or not efficient or both, in order to allow small, desk-top sized or wearable blood purification systems.

Removing toxins from blood and tissue via electrical activated oxidation has been described already in 1975 e.g. in US3878564. In 1982, in particular US4473449, such a process has been described for the regeneration of dialysate fluid using Pt, Ti with TiO2, SnO2 or RuO2 coatings as electrode materials. This process has never been applied nor practiced in blood purification, most likely because the process produces unwanted oxidation products from partly oxidized aminoacids and proteins.

Removing toxins via an electrosorption device has been well described in patent EP2092944 published in 2009. Athough the proposed technology is regarded as an important step forward in blood purification, the removal of urea is still problematic and reguires a relatively high volume and mass of sorbents.

It is an object of the present invention to overcome the problems associated with the devices of the prior art and to provide a compact and efficient sorption-filter and decomposition system for use in hemodialysis, peritoneal dialysis systems and -more genericly- for use in blood purification systems such as a wearable artificial kidney, artificial liver, artficial lung etc.

In similar form such a system is applicable for purification of other fluids such as the purification of water, waste water treatment and purification of e.g. aquarium water.

### SUMMARY OF THE INVENTION

This problem is solved according to the invention by providing a device comprising:
i) a housing having an inlet for entry of water, dialysate fluid, blood or blood plasma or ultrafiltrate into said housing, an outlet for the removal of purified water, dialysate fluid, blood or blood plasma or ultrafiltrate and excess fluid from said housing, and a conduit connecting said inlet with said outlet
ii) an electrocatalytic decomposition filter for removing urea, urate, ammonia, creatinine and other components containing amine(NHₓ)-groups from the water, dialysate fluid, blood, bloodplasma or ultrafiltrate said electrocatalytic decomposition filter being contained in said conduit such that said water, dialysate fluid, blood or blood plasma or ultrafiltrate must pass through said electrocatalytic decomposition filter when flowing from said inlet to said outlet and said electrocatalytic decomposition filter comprising:
   a) a set of electrodes made from or coated with a catalytic material for the decomposition of urea, urate, ammonia, creatinine and other components with amine-groups or a set of electrodes that are in electrical contact with materials with electro-catalytic active surfaces
   b) a power source to provide the electrodes with an electrical charge and electrical current in order to initiate and sustain the catalytic breakdown .
   c) an electronic device that enables a periodic switch in the polarity of the electrodes in order to prevent unwanted deposition of molecules on the electrodes iii) an electrosorption filter for removing toxins, toxic solutes, toxic small and middle-sized molecules and protein bound toxins from the water, dialysate fluid, blood, bloodplasma or ultrafiltrate said electrosorption filter being contained in said conduit such that said water, dialysate fluid, blood or blood plasma or ultrafiltrate must pass through said electrosorption filter when flowing from said inlet to said outlet and said electrosorption filter comprising:
      a) a sorption material selected from the group consisting of:
         1) a nanostructured sorption material;
         2) a porous polymer matrix, wherein the pores in said matrix are of a size that allows the entry into said matrix of toxic substances sought to be removed from said liquid and/or preventing the entry into said matrix of substances not sought to be removed from said liquid; and
         3) a nanostructured sorption material captured in a porous polymer matrix, wherein the pores in said porous polymer matrix are of a size that allows the entry into said matrix of substances sought to be removed from said liquid while preventing the escape of said nanostructured sorption material and/or preventing the entry into said matrix of substances not sought to be removed from said liquid; said sorption filter being contained in a housing;
      b) a set of electrodes coated with or in electrical contact with the sorption material;
      c) a power source to provide the electrodes with an electrical charge in order to generate an electrical field strength in the sorption medium.

Said toxic substances are preferably selected from potassium, phosphate, urea, uric acid, ammonia, creatinine, beta2-microglobulin (2M), and albumin-bound toxins such as paramino hyppuric acid, p-cresol, indoxyl sulphate, CMPF and bilirubin The electrosorption filter combines nanostructured sorbent materials with a high specific and selective surface area together with additional electric surface charging delivered by an external power source. This combination enables a very efficient, highly effective and therefore small sized sorption system. The combination with a filter with electrodes that provide catalytic decomposition of urea, urate, ammonia, creatinine and other components with amine-groups provide that these toxins can be removed via gasification in stead of absorption, reducing the amount of sorbents that are needed considerably. This enfavours a strong reduction in size of the device. Possibly unwanted oxidation products arising from this electrocatalytic decomposition process such as partly oxidized aminoacids, peptides and proteins as well as unwanted residues from the electrocatalytic decomposition such as chloramines and chlorine are being removed effectively by the electrosorption filter.

The electrosorption filter and the electrocatalytic decomposition filter therefore operate in a very synergetic mode: the electrocatalytic decomposition filter enables a strong reduction of the volume of the sorbents in the electrosorption filter, whereas the electrosorption filter removes the resulting uremic toxins and unwanted by-products from the electrocatalytic decomposition filter.

The electrosorption filter and the electrocatalytic decomposition filter can ideally be integrated into one electrosorption/decomposition system wherein the electrodes of the electrosorption filter are made of electrocatalytic decomposition material. The electrosorption and electrocatalytic breakdown process are than combined. This allows a further reduction in size and weight. Another important feature of such a combined setup is that the sorption materials are continuously exposed to the electro-catalytic decomposition process. This prevents that sorption materials are being clogged with organic deposites such as proteins.

The small size and subsequent low weight allows for a small, desk-top size system and even a wearable device for water treatment, blood filtration or hemodialysis or peritoneal dialysis is feasible.

Said electrosorption filter comprises an absorbing, adsorption, ion-exchange and/or surface crystallisation material with very small nano sized particles or pores offering a large specific surface area in combination with a high chemical surface activity like activated carbon, nanoclays, nanocrystalline hydrotalcites, nanoporous silica's, nanoporous or layered alumina silicates (like zeolites), nanoporous metal oxides and metal hydroxides, metal organic frameworks, zeolite imidazolate frameworks, nanosized and /or activated graphite, cyclo-dextrines, crystallisation seeds, a highly porous matrix material with again a large specific surface area, with tuneable porosity and a high specific chemical activity like carboxymethyl cellulose and like a biopolymer such as oxidized starch modified with functional groups for specific absorption or combinations thereof.

Said electrocatalytic decomposition filter comprises a set of electrodes made from or coated with a catalytic material such as Pt, Ni, Ti, Sn, Ir, Ta and Ru and their oxides TiO2, RuO2, RiO2, SnO2, Ta2O3, NiO or hydroxide forms such as and NiOOH and Ni(OH)2 or mixtures thereof. These materials enable decomposition via electro-oxidation of urea, ureate, ammonia, creatinine and other components with amine groups (like proteins and peptides) into gases such as N2, CO2 and H2 according to the following reaction:

CO(NH₂)₂ (s) + H₂O (s) > N₂ (g) + CO₂ (g) + 3H₂ (g)

In other embodiment the electrocatalytic decomposition filter comprises a set of electrodes in electrical contact with materials with electrocatalytic surface activity such as sorbents coated with Pt, Ni, Ti, Sn, Ir, Ta and Ru and their oxides TiO2, RuO2, RiO2, SnO2, Ta2O3, NiO or hydroxide forms such as and NiOOH and Ni(OH)2 or mixtures thereof.

An embodiment of the device according to the invention is characterized in that the nanostructured sorption material is selected from the group consisting of:
i) nanoparticles or nanocrystalline materials, preferably metal silicates such as alumina silicates (like zeolites), nanoclays, preferably an exfoliated nanoclay, metal hydroxides, metaloxyhydroxides, layered double hydroxides like a nano-hydrotalcite or pure metaloxide nanoparticles;
ii) nanoporous materials, preferable selected from zeolites, mesoporous systems, carbonaceous nanomaterials and metal organic frameworks;
iii) nanocomposites;
iv) nanofibers, and
v) any combination of the above.

In another preferred embodiment of a device of the present invention said porous polymer matrix is based on a cross-linked polymer and/or a charged polymer and/or a polymer modified with specific functional groups for specific absorption.

In yet another preferred embodiment said polymer is a biopolymer selected from carbohydrates and proteins.

In still another preferred embodiment said carbohydrate is an oxidized crosslinked starch.

In still another preferred embodiment said carbohydrate is a carboxymethyl cellulose.

In another preferred embodiment the sorption material is provided in the form of a permanent coating on the electrode, a replaceable gel or replaceable dried granules having a mean size over the range 250 microns to 1500 microns based on the size in dried form.

Preferably the pores in said matrix are of a size that prevents the entry into said matrix of albumin and other useful blood components such as transferrin and vitamin B 12.

A further embodiment of the device according to the invention is characterized in that said electrosorption filter is provided in the form of a cartridge with said electrodes coated with said sorption material or with said electrodes surrounded by a porous envelop containing said sorption material.

Another embodiment of the device according to the invention is characterized in that said electrosorption filter is combined with a permeable envelop to form a filter pad comprising an envelope surrounding a filter pad contents, wherein the envelop of said pad comprises a permeable membrane and the contents of said pad comprise said sorption material.

Another embodiment of the device according to the invention is characterized in that said electrosorption filter is combined with an electrocatalytic decomposition filter with electrodes for the electrocatalytic decomposition and gasification of urea, urate, ammonia, creatinine and other components containing amine groups, wherein the electrodes are being activated with a voltage of 0-20V, preferably with a voltage of 0,5-4 V. A low voltage limits direct hydrolysis of water that otherwise will consume additional energy.

A further embodiment of the device according to the invention is characterized in that said device further comprises a plasmafilter for separating patient blood plasma from patient blood cells.

A further embodiment of the device according to the invention is characterized in that said device further comprises a hemofilter for separating blood ultrafiltrate from the patients blood, wherein the ultrafiltrate is being separated from blood cells, platelets and large (typically > 50 kDa) molecules such as albumine, protein S and C and LDL-cholesterol.

In this embodiment said sorption-filter is combined with a plasma- or hemofilter system in order to form an artifical kidney device for the removal of toxic substances from the blood from a patient, comprising a plasmafilter for separating patient blood plasma from patient blood cells, or a hemofilter to separate blood ultrafiltrate from the patient blood, electrodes in combination with a sorption-filter for removing toxins, toxic small- and middle-sized molecules and protein bound toxins from the patient blood plasma and optionally to supplement the blood with at least one substance selected from the group consisting of vitamins, minerals, anticoagulants, anti microbial agents and other medicaments and electrodes made from or coated with catalytic material for the decomposition and gasification of urea, urate, ammonia and other components with amine -groups.

In case of blood filtration, a plasmafilter for separating patient's blood plasma from the blood or a hemofilter for separating patient's blood ultrafiltrate from the blood is used followed by the electrosorption and decomposition filter for removing toxic ionic solutes, toxic small- and middle-sized molecules and protein bound toxins from the patient's blood plasma or ultrafiltrate.

In yet another preferred embodiment said device is part of a hemodialysis or peritoneal dialysis system in order to improve the absorption capacity of the dialysis fluid or to regenerate the dialysate insitu via said electrosorption and decomposition filter and hereby mimimizing the dialysate fluid volume and the dimensions of the dialysate system, thereby allowing the dialysis system to be wearable.

In yet another preferred embodiment said dialysis system is a wearable dialysis system.

In a further preferred embodiment said plasmafilter or hemofilter comprises at least a further outlet for recovery of patient blood cells.

Preferably said plasmafilter or hemofilter and said electrosorption and decomposition filter are combined to form the filter pad wherein said permeable membrane is formed by said plasma filter or hemofilter.

A still further embodiment of the device according to the invention is characterized in that said device further comprises an electrosorption filter for removing toxins, small and middle-sized molecules from the patient blood plasma.

A still further embodiment of the device according to the invention is characterized in that said device further comprises an electrocatalytic decomposition filter for removing urea, urate, ammonia, creatinine and other, mainly amine group containing molecules from the patient blood plasma or ultrafiltrate via decomposition into small chemical species and subsequent gasification.

Yet a further embodiment of the device according to the invention is characterized in that said device further comprises means for supplementing said dialysate fluid and/or said (purified) blood plasma or ultrafiltrate with at least one substance selected from the group consisting of vitamins, minerals, anticoagulants, anti microbial agents and other medicaments.

Preferably said device further comprises ion exchange systems and materials in combination with the electrical charge from the electrodes.

A further embodiment of the device according to the invention is characterized in that the performance of the sorption filter is being monitored with a sensor system measuring the quality of the dialysate, blood or bloodplasma or ultrafiltrate in the outlet.

Preferably, the sorptionmaterials are being regenerated with a regeneration fluid in combination with a reversed voltage mode on the electrodes.

A still further embodiment of the device according to the invention is characterized in that the device comprises a control unit controlling the electrocatalytic decomposition rate and the sorption activity of the sorption material by a voltage between 0-200V of an external power source in order ensure a field strength in the sorption medium of typically 10-20 V/cm.

In order to enhance and to regulate the sorption capacity, the sorption material is connected via electrodes to an external DC electrical power source such as a battery or a rectifier. Hereby, the electrical surface charge of sorption material can be externally controlled. A voltage in the range of 0-3 V, with typical values of 0,5-1,5 V is used. The voltage on the electrodes for catalytic decomposition or urea, urate, ammonia, creatinine and other amine group -containing components is in the range of 0-20 V, with typical values of 0,5-4,0 V. A higher voltage will increase the rate of electrolysis of the water with negative effects on the energy consumption.

By regulating the voltage and electrical current of the power source, the surface charge of the electrodes and the sorption material can be augmented, maintained, diminished or reversed. Since the surface charge is the major driving force for molecular adsorption and binding, the capacity of the sorption material can be increased or decreased depending on the need.

The sorption material can also be regenerated by operating the device in reversed mode, with a voltage reversal on the electrodes leading to a repulsion of bound toxins.

The regeneration of sorbents can also be achieved by flushing the sorbents with a regeneration fluid that will promote reversed ion-exchange and subsequent release of toxins. Such a regeneration fluid may consists of a water solution with specific salts, such as NaCl solution (typical concentration 1-10 wt%) or other sodium salts. The regeneration fluid might also contain other salts (preferably chlorides) from calcium, magnesium, iron or other components such as vitamines, EPO and medicine. Hereby the sorbents cannot only be regenerated but also be preloaded with components. These components will then be released to the blood system in normal use. This preloading is also of use in order to neutralise unwanted uptake of specific components by the sorbents.

The regeneration of sorbents can also be promoted by a reversed electrode mode operation in combination with a regeneration fluid.

Reversal of the voltage on the electrodes for the electrocatalytic decomposition is needed in order avoid inactivation due to electro-deposition of components from the fluid. The frequency for voltage reversal is not very critical and can vary from switching every second to switching every 10-20 minutes. However it is recommended to keep the switching time within a range of 10-100 seconds.

Preferably the voltage of the external source is electronically regulated depending on the reading of the sensor system.

The voltage of the external source can be reversed into opposite values in order to repell and to release toxins, unwanted deposits and to regenerate the sorption materials.

The sorption electrodes are preferably planar, circular, tubular or thread electrodes made from Ag, Ti, stainless steel, Cr or other blood compatible materials or with a coating hereof.

The catalytic electrodes are preferably planar, circular, tubular or thread electrodes made from Pt, Ni, Ti, Ir, Sn, Ta and Ru and their oxides or hydroxide form such as TiO2, RuO2, RiO2, SnO2, Ta2O3, NiO and NiOOH and Ni(OH)2 or mixtures thereof.

In a preferred embodiment said artificial kidney system is a system that improves the clearance performance of existing hemodialysis and peritoneal systems.

In a preferred embodiment said artificial kidney system is a system that eliminates the use of a dialysate fluid in an existing hemodialysis system.

In a preferred embodiment said artificial kidney system is a system that uses a small volume of dialysate fluid that is being regenerated and recirculated.

In a preferred embodiment said artificial kidney system is a wearable device.

The present invention provides a device for the removal of toxic substances from a water stream, waste water or biofluid such as blood, blood plasma, ultrafiltrate or dialysate, said device comprising:
i) a compartment with a set-up of electrodes and sorption materials allowing removal of toxins via electrocatalytic decomposition and gasification and via extraction of toxins from the fluid in the compartment by electrosorption, and also desorption of toxins by reversed electrical operation,
ii) electrodes that are coated with a catalytic material for the decomposition and gasification of urea, urate, amonia, creatinine and other components containing amine groups or electrodes that are in electrical contact with materials in the said compartment with similar electrocatalytic surface activity, allowing this material to be activated or de-activated by the electrical charge delivered by the electrodes and the external power source,
iii) electrodes that are coated with a sorption material or electrodes that are in electrical contact with sorption materials in the said compartment, allowing the sorption material to be activated or de-activated by the electrical charge delivered by the electrodes and the external power source,
iv) a sorption material for removing toxins, small and middle-sized molecules from the biofluids, said sorption filter comprising a sorption material selected from the group consisting of:
   - a nanostructured sorption material offering a high specific surface area with specific (ab/ad)sorption affinity;
   - a porous polymer matrix, wherein the pores in said matrix are of a size that allows the entry into said matrix of toxic substances sought to be removed from said liquid and/or preventing the entry into said matrix of substances not sought to be removed from said liquid; and
   - a nanostructured sorption material captured in a porous polymer matrix, wherein the pores in said porous polymer matrix are of a size that allows the entry into said matrix of substances sought to be removed from said liquid while preventing the escape of said nanostructured sorption material and/or preventing the entry into said matrix of substances not sought to be removed from said liquid.
v) an inlet for entry of the water stream, waste water or biofluid into said device,
vi) an outlet for the removal of purified water stream, waste water or biofluid from the said device, and
vii) a conduit connecting said inlet with said outlet and holding a compartment with said electrodes and said sorption filter such that the water stream, waste water or biofluids must pass through said compartment with electrodes and sorption material when flowing from said inlet to said outlet.

In a preferred embodiment of a device of the present invention said device comprises a compartment with:
a) planar, circular, tubular or thread electrodes made from Pt, Ni, Ti, Ir, Sn, Ta and Ru, said electrodes being coated with their oxides or hydroxide form such as TiO2, RuO2, RiO2, SnO2, Ta2O3, NiO and NiOOH and Ni(OH)2,
b) planar, circular, tubular or thread electrodes made from Ag, Ti, stainless steel, Cr or other blood compatible materials or with a coating hereof or the electrodes as decribed in a), said electrodes being coated with or in electrical contact with a porous, preferably nanostructured sorption material,
c) an electrical control unit capable of controlling the electrical voltage on each of the electrodes,
d) a sensor unit capable of measuring the toxin concentration in the cleaned (waste) water or biofluids, e.g. via an electrical conductivity measurement,
e) a pumping unit to ensure sufficient water or biofluid flow through the compartment, in case of bloodplasma, dialysate or ultrafiltrate of a renal patient this is typically 20-100 ml/min,
f) optional filters such a plasma filter or a hemofilter for blood - blood plasma separation, blood - ultrafiltrate separation or blood-dialysate exchange.

### BRIEF DESCRIPTION OF THE DRAWINGS

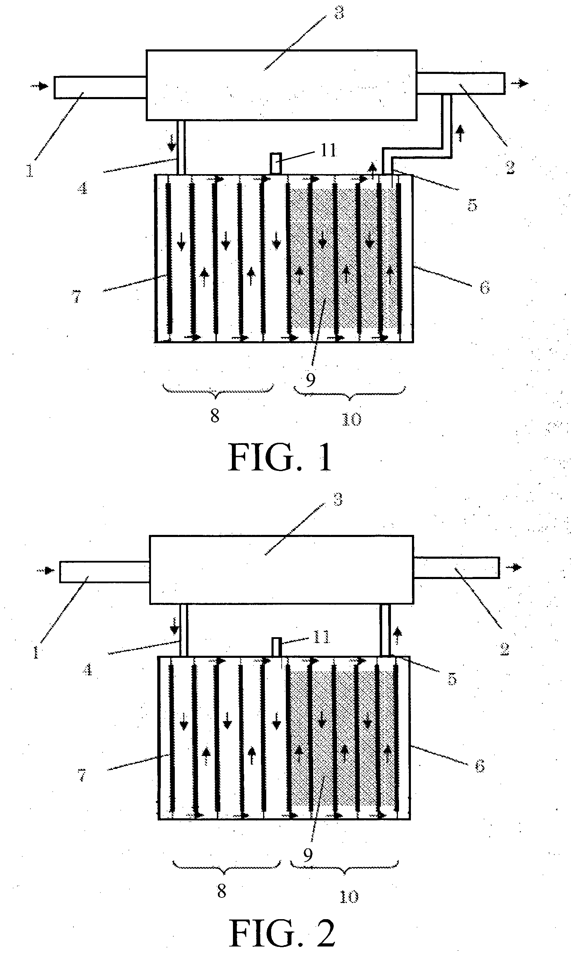

Figure 1 shows a cross-sectional presentation of a device according to the invention with catalytic electrodes for decomposition and degasification of toxins with NHₓ groups such as urea and creatinine and electrosorption electrodes surrounded with nanostructured sorption materials for subsequent adsorption of other toxins as small solutes, middle molecules and protein bound toxins. The device is set for blood purification via extraction of ultrafiltrate via a hemofilter. The purified ultrafiltrate is given back to the patient.
Figure 2 shows a similar cross-sectional presentation of a such a device, but now based on a dialysate system. Here the dialysate is being continuously purified and regenerated by the same electrosorption and decomposition filter.

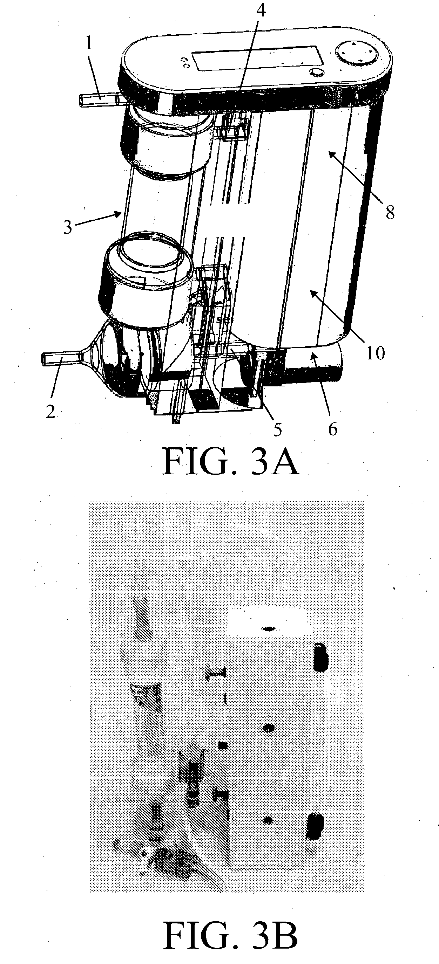

Figures 3A and 3B show an engineering drawing of wearable device incorporating an hemofilter and an integrated electrosorption and decomposition filter system. Thanks to the nanostructured sorption materials in combination with the electrocatalytic decomposition, a very small device becomes now very feasible.

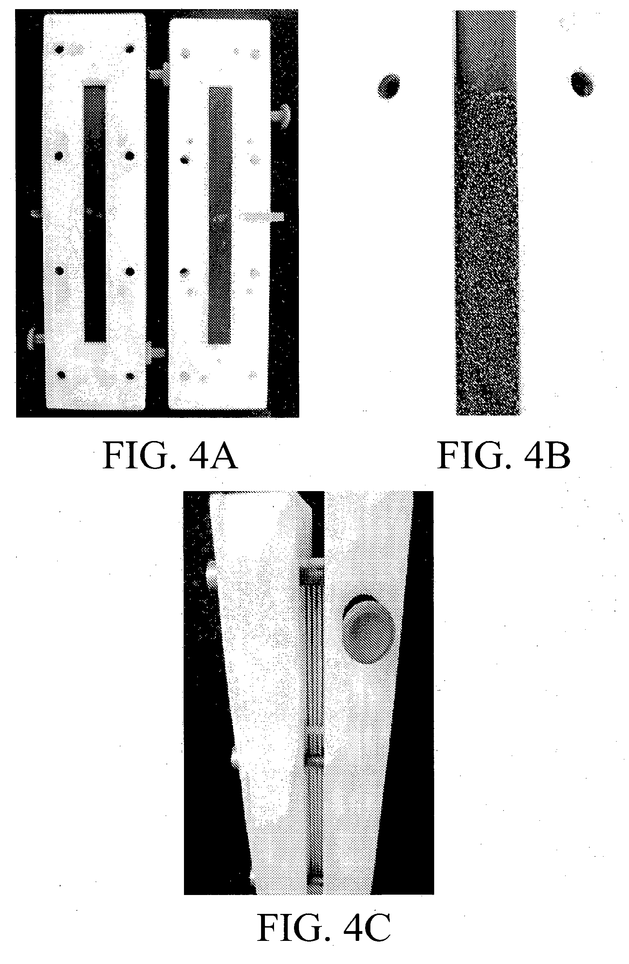

Figures 4A, 4B and 4C show prototyped components of an electrosorption and decomposition unit with catalytic electrodes (left), an electrosorption chamber partly filled with adsorbents (middle) and a complete unit with stacked electrodes for catalytic decomposition in combination with electrosorption. This allows for a very small and wearable device featuring a 24hrs/day, continuous cleansing operation. This results in a much better clearance of the blood improving the health condition and life expectancy of renal patients very considerably.

### DETAILED DESCRIPTION OF THE INVENTION

The term "sorption" as used herein, refers to both adsorption and absorption. Adsorption is a process that occurs when a gas or liquid or solute (called adsorbate) accumulates on the surface of a solid or more rarely a liquid (adsorbent), forming a molecular or atomic film (adsorbate). It is different from absorption, where a substance diffuses into a liquid or solid to form a "solution". The term sorption encompasses both processes, while desorption is the reverse process.

The term "small-sized molecules", as used herein, refers to molecules with a molecular weight lower than 500 Da, such as uric acid, urea, guanidine, ADMA, creatinine.

The term "middle-sized molecules", as used herein, refers to molecules with a molecular weight between 500 Da and 5000 Da, such as end products from peptides and lipids, amines, amino acids, protein bound compounds, cytokines, leptins, microglobulins and some hormones.

The term "nanoporous materials" refers to materials having pores that are by definition roughly in the nanometre range, that is between 1x10⁻⁷ and 0.2x10⁻⁹ m and includes reference to the 3 categories set out by IUPAC of microporous materials (such as zeolites) having pore sizes of 0.2-2nm; mesoporous materials having pore sizes of 2-50nm; and macroporous materials having pore sizes of 50-1000nm.

The term "ionic solutes", as used herein, refers to components such as phosphates, sulphates, carbon hydrates, chlorides, ammonia, potassium, calcium, sodium.

"Nano sized" as used herein, refers to a size of approximately 1-1000 nm, more preferably 1-100 nm.

The term "electrosorption" refers to a process where ionic solutes and charged molecules in a solution are being adsorpted onto the surface of a sorbent with the help of an additional electrical surface charge on the sorbent provided by an electrical power source.

The term "electrosorption filter" refers to a filter system comprising a sorbent that can be electrically charged or discharged with an external power source via electrodes connected to the sorbent material.

The term "catalytic" as used herein, refers to a process that specific chemical reactions are being promoted when in contact with another material due to specific intermolecular interactions.

The term "electrocatalytic" as used herein refers to a catalytic chemical reaction that is being initiated via electrical activation.

The term "decomposition" as used herein refers to a chemical reaction where a component is broken down to its smaller constituents.

The term "gasification" as used herein refers to a chemical reaction where the component is being gasified and released from the system.

The term "electrocatalytic decomposition" as used herein refers to a process where a chemical component is being broken down into smaller chemical species, preferrably gaseous species, via electrocatalytic oxidation.

The term "electrocatalytic decomposition filter" as used herein refers to a filter system comprising electrodes that remove toxins via electrocatalytic activated decomposition of these toxins and subsequent gasification.

The term "electrosorption-decomposition filter" as used herein refers to a filter system that combines an electrosorption filter and an electrocatalytic decomposition filter.

### The purification device holding an electrosorption and decomposition filter package

A device of the present invention can take the form of an electrosorption and decomposition filter package that is placed in the dialysis fluid system of a hemodialysis or peritoneal dialysis system, enabling the removal of toxins from the dialysis fluid. The electrosorption and decomposition filter continuously purifies the dialysate fluid, keeping the the toxin concentration in the dialysis fluid low, resulting in an improvement of the hemodialysis and peritoneal dialysis efficiency, typically with 100%, and reduces the consumption of dialysis fluid needed dramatically, ideally down to 1-10 litres. An additional and optional function of the sorption filter is to release ingredients for supplementing of the blood such as calcium, vitamin A, C and B12, anti-coagulation agents, anti microbial agents, minerals, specific medicaments etc. This option will simplify the operation of existing hemodialysis and peritoneal dialysis systems and will reduce the chance on occurring infections in the peritoneal dialysis system.

A device of the present invention can take the form of wearable peritoneal dialysis system wherein the electrosorption and decomposition filter package is placed in a wearable peritoneal dialysis system. Due to the continuous filtering of the electrosorption and decomposition filter, the volume of dialysate fluid can be reduced to typically 1-2 litres. The wearable peritoneal dialysis device comprises a tubular access system to the abdominal cavity and a unit comprising a fluid pump, power, sensors, electronic control, a facility to place and replace said electrosorption and decomposition filter package, typically on a daily basis and a system to dispose off excess water. An additional and optional function of the sorption filter is to release ingredients for supplementing the blood, such as calcium, vitamin A, anti-coagulation agents, anti microbial agents, minerals, specific medicaments etc. This option will enhance the operation of the peritoneal dialysis system and will reduce the chance on occurring infections.

A device of the present invention can take the form of wearable hemodialysis system wherein the electrosorption and decomposition filter system is placed in a wearable hemodialysis system. Thanks to the continuous filtering of the electrosorption and decomposition filter, the volume of dialysate fluid can be reduced to typically 200-400 ml. The wearable hemodialysis device comprises a vascular access tubing system and a unit comprising a small hemofilter system, fluid pump, power, sensors, electronic control, a facility to place and replace said electrosorption and decomposition filter package, typically on a daily-to-weekly basis, and a system to dispose off excess water. An additional and optional function of the electrosorption filter is to release ingredients for supplementing the blood such as calcium, vitamin A, anti-coagulation agents, anti microbial agents, minerals, specific medicaments etc. This option will simplify the operation of the hemodialysis system and will reduce the chance on occurring infections.

A device of the present invention can take the form of a wearable or desktop sized artificial kidney based on blood plasmafiltration or blood ultrafiltration combined with electrosorption and decomposition filtering. In such an embodiment, the blood plasma filtration step will performed by a special plasma filter, or the ultrafiltrate will be extracted via a special hemofilter, with a relative large pore size, that separates blood from plasma or the ultrafiltrate, allowing toxic solutes, small/middle molecules and protein bound toxins to pass with the plasma or ultrafiltrate into the compartment with the electrosorption-decomposition filter package for cleansing. Via an additional hemofilter with a smaller pore size, excess water can be removed from the blood plasma or ultrafiltrate preventing loss of albumin. The cleansed blood plasma or ultrafiltrate is then re-entered into the bloodstream. It will be understood that in such an embodiment, the device further comprises the necessary tubing, vascular access and feedback systems, pumping, electronics, sensors, power packs and other requirements. However, these are not essential to the present invention. An advantage of the artificial kidney device is that no dialysis fluid will be needed. In a preferred embodiment of this device, the plasma, ultrafiltrate or hemofilter and the electrosorption and decomposition filter are being combined to form a filter package, said filter package comprising an envelop, made from a hemofilter material, surrounding a filter material and electrodes. A representative embodiment of an artificial kidney device of the present invention is depicted in Figures 1 and 2.

The device as depicted in Fig. 1 is a cross-sectional presentation of a device for blood purification. Blood from a patient is fed via blood inlet 1 to the plasma or a hemofilter 3. Blood is fed back to the patient via outlet 2. Plasma or ultrafiltrate is extracted at conduct 4 and fed into the electrosorption and decomposition unit 6. The plasma or ultrafiltrate enters the electrocatalytic decomposition section 8 with catalytic electrodes 7. Here urea, urate, ammonia, creatinine and other toxins with amine-groups are being decomposed and degased via gasoutlet 11. The voltage over each pair of electrodes can be as high as 20 V but in order to limit hydrolysis, the voltage difference should be kept low, typically below 4 V. Other toxins such as the small solutes potassium and phosphate as well as toxic middle molecules as beta2microglobulin and the protein bound toxins such as p-cresol, indoxylsulphate and hyppuric acid are adsorbed in the electrosorption section 10 where sorbents 9 can be activated and cleansed via electrodes 7. The positively charged electrodes, with an electrical charge of 0-200 Volt, will attract and bind the negatively charged toxins such as phosphates, beta2microglobulin and most of the other toxic proteins. The negatively charged counter electrodes will attract and bind the positively charges toxins such as potassium and ammonia. The electrical driving force for the attraction is the electrical field strength in the sorption medium. A field strength of typically 10-20 V/cm has proven to be quite effective in accelerating the toxins and in promoting the binding. The actual voltage to be applied on the electrodes will therefore depend on the electrode distance. In order to prevent hydrolysis of the fluid, a low voltage is recommend, typically 1.2-1.4 V. Such a low voltage is only effective if the electrode distance is small, typically in the range of 0.5-1.0 mm. However, if hydrolysis or other side effects of a high voltage in the fluid can be accepted, an electrode distance of several cm's is possible.

The nanostructured sorption materials offer a very large surface area and chemical activity to bind a high load of toxins, typically in the range of 10-50% on weight basis. This purification mode can be operated until the sorption material is saturated with toxins. This depends on the amount of sorbent used. A representative embodiment will comprise 50-150 grams of sorbent, allowing a continuous purification mode to last 12-24 hours. The device can be turned into a reverse mode . The device is set into reverse mode by switching the external voltage into its opposite sign in combination with a regeneration fluid With the reversed voltage, the sorption function changes into a repellent function and the toxins are enforced to unbind and to leave sorbent surface. Hereby the sorbent is being cleaned and regenerated. The released toxins in the compartment are removed by disposing the regeneration fluid plus some ultrafiltrate fluid. The volume of the disposed ultrafiltrate is typically around 1-1,5 L per day, the normal amount of excess fluid to be released by a human.

In figure 2 a similar scheme is depicted but here the system is adapted for a (wearable) hemodialysis unit. Blood is cleansed via a dialysate fluid, typically 300-500ml, that is being fed to the hemofilter 3. The dialysate is continuously purified with the electrosorption and catalytic decomposition unit.

In Fig 3 an engineering drawing is shown of a complete wearable artficial kidney device next to a photo of a laboratory set-up. It comprises a hemofilter in order to exchange toxins from the blood to the dialysate and to extract ultrafiltrate. The electrosorption and decomposition unit continuously removes the toxins from the dialysate.

Prototyped components are shown in Fig 4, with the electrocatalytic electrodes (left), electrode compartments filled with adsorbents (middle) and a complete unit with stacked electrode chambers for decomposition and electrosorption.

The hemofilter in a device of the present invention is preferably a commercially available hemofilter (e.g. such as produced by Gambro GmbH, Hechingen, Germany or Membrana GmbH, Wuppertal, Germany).

In an alternative embodiment the electrosorption and decomposition filter in a device of the invention may comprise an inlet for receiving patient blood plasma or ultrafiltrate exiting said plasmafilter or hemofilter, and at least one outlet for recovery of purified blood plasma or ultrafiltrate.

A device of the present invention may, in any embodiment, further comprise means for supplementing the (purified) blood plasma or dialysate fluid with at least one substance selected from the group consisting of vitamins such as vitamins A,C and B 12; minerals such as calcium, sodium and potassium; anticoagulants; anti microbial agents and other medicaments.

A device of the present invention may, in any embodiment, further comprise means for selective sorption of middle molecules, vitamins and minerals such as calcium, sodium and potassium. The sorption materials as described (smectites, nanoclay, layered double hydroxides, hydrotalcites, crystallisation seeds, metal organic frameworks, modified biopolymers etc.) are therefore loaded with a certain amount of minerals, vitamins and can only absorb a designated amount.

A device of the present invention may, in any embodiment, further comprise means for selective sorption of middle molecules, vitamins and minerals such as calcium, sodium and potassium in the sorption pad via osmotic differences between blood and dialysate fluid in combination with the filterpad. The blood plasma or ultrafiltrate in this variant essentially remains in circulation and does not enter as a whole the filter pad, but is being cleaned from toxic substances and is being supplemented with nutrients and other vital substances via the dialysate fluid in combination with the sorption unit.

Optionally, the device or filter pad may comprise ion exchange systems.

In another aspect, the present invention provides a method for removing toxic substances from blood, comprising using a device according to the present invention.

In another aspect, the present invention provides a method for removing toxic substances from hemodialysis or peritoneal dialysis fluids, comprising using a device according to the present invention.

In another aspect, the present invention provides a method for removing substances from other fluids such as water, e.g. for making drinking water or purifying aquarium water, and for purifying chemicals used in industrial processes as wel as removing substances from other biofluids such as urine, milk, bio-analytical fluids, comprising using a device according to the present invention.

The electrosorption and decomposition filter in a device of the present invention may take the form of a filter pad, consisting of a rigid or flexible container comprising the sorption materials and electrodes.

The electrosorption and decomposition filter in a device of the present invention may take the form of a filter cartridge, consisting of a rigid or flexible container comprising a set of built-in electrodes and a replaceble filter pad with sorption materials.

This, in another aspect, the present invention may take the form of a device with built-in electrodes connected to a power supply such as a battery or a rectifier, holding an electrosorption and decomposition filter cartridge in contact with these electrodes and comprising a blood plasma separator or hemofilter and a sorption filter pad with sorption materials for extracting ionic solutes and small and middel sized molecules from the blood plasm, ultrafiltrate or dialysate.

### The nanostructured sorption material comprised in the electrosorption filter

The nanostructured material exhibits sorption capacity of various substances, based on ion-exchange, surface adsorption activity and/or surface nucleation (surface crystallisation).

The sorption material is preferably functionalized, such as to exhibit improved sorbing properties of toxic substances such as urea as compared to the non-functionalized material. The sorption material in the present invention is a nanomaterial, meaning that the material contains particles or contains pores with a size of preferably 100 nanometres or less in order create a large specific surface area.

A suitable nanostructured sorption material will be based on a blend of nanomaterial components, each component offering a specific functionality. Depending on the individual needs, a selection is made out of the following nanomaterial classes and components:
i) Layered double hydroxides (LDH's) or so called anionic clays or hydrotalcite-like compounds, comprising an unusual class of layered materials with positively charged layers and charge balancing anions located in the interlayer region. Hydrotalcite-like compounds (HT) can be represented by the following formula: [Mg₁₋ₓ Alₓ (OH)₂]^{x+} [A_{x/n}ⁿ⁻. mH₂0]^{x-}, wherein 0 x < 0.33, and Aⁿ⁻ is an exchangeable anion having a valence of n. These compounds are similar to the mineral hydrotalcite, Mg₆Al₂(OH)₁₆CO₃.4H₂0_{.} LDH's may have different cations, such as Mg, Mn, Fe, Co, Ni, Cu and/or Zn as divalent cations, and Al, Mn, Fe, Co, Ni, Cr and/or Ga as trivalent cations. Particularly preferred hydrotalcites are Mg₂Fe(OH)₆.OH and Mg.OH. It should be noted that hydrotalcites used in aspects of the present invention need not be layered, but may be provided in the form of nanocrystalline materials.
ii) Nanoclays, phyllosilicates, or layered silicates have negatively charged layers and cations in the interlayer spaces offering ion-exchange, adsorption and surface nucleating activity. Preferred are smectite-like clay minerals, such as montmorillonite, saponite, hectorite, fluorohectorite, beidellite, nontronite, vermiculite, halloysite and stevensite. Very suitable are clays based on magnesium and/or alumina silicate layers with a cation exchange capacity between 50 and 200 milliequivalents per 100 gram. Exemplary nanoclays are available from e.g. Nanocore (USA) and Sudchemie (Germany)
iii) Nanocrystalline metal oxides and metal hydroxides of Mg, Sr, Ba, Ca, Ti, Zr, Fe, V, Cr, Co, Y, Mn, Ni, Cu, Al, Si, Zn, Ag, Au, Mo, Sb, Ce and mixtures thereof e.g. known from WO 2007/051145. The nanocrystalline materials preferably present crystallite sizes of less than about 25 nm, more preferably less than 20 nm, and most preferably less than 10 nm with a surface area of at least about 100 m²/g, more preferably at least about 300 m²/g, and most preferably from about 700 m²/g and more. Exemplary nanocrystalline materials are available from NanoScale Materials, Inc., Manhattan, Kansas, under the name NanoActive®.
iv) Nanoporous materials, characterized by the molecular assembly of structures consisting of nanometer-sized cavities or pores. Nanoporous materials for use in the present invention may include active carbon, nanoporous silica's, nanoporous alumina silicates such as zeolites. Very suitable nanoporous materials for use in the present invention are metal organic frameworks (MOFs). Metal organic frameworks are hybrid materials where metal ions or small nano-clusters are linked into one-, two- or three-dimensional structures by multi-functional organic linkers as described, for example, in US 5,648,508 and US 6,893,564. In many cases it is possible to obtain open micro- and mesoporous structures having high porosities and specific surface areas of even above 5000 m2/g. Such open, porous, structures are very suitable for use as nanostructured adsorbents in the present invention. The metal organic frameworks for use in the present invention may be based on Mg, Sr, Ba, Ca, Ti, Zr, Fe, V, Cr, Co, Y, Mn, Ni, Cu, Al, Si, Zn, Ag, Au, Mo, Sb, Ce or any other metal that provides good adsorption characteristics. The preferred metals in applications for dialysate fluid or blood (plasma) purification in nanostructured materials used in aspects of the present invention are preferably based on metals such as Fe, Ti and Mg. Tectosilicates and tetrasilicates are another class of synthetic or natural aluminosilicates that are crystalline porous nanostructures having long-range crystalline order with pore sizes that may be varied from about 2 Å to 200 Å (Angstroms).
v) Carbonaceous nanomaterials, suitable for use in aspects of the present invention include fullerenes, carbon nanoparticles, diamondoids, porous carbons, graphites, microporous hollow carbon fibers, single-walled nanotubes and multi-walled nanotubes.

A sorbent of the present invention, comprising a nanostructured sorption material captured in a porous polymer matrix, may be prepared by providing the nanostructured sorption material and the porous polymer matrix separately and combining the two such that the nanostructured sorption material is inserted in the porous polymer matrix. This may for instance occur by instilling the polymer matrix with a suspension of the nanostructured material.

Alternatively, in order to obtain a polymer matrix having a nanostructured material dispersed therein, one may prepare the matrix having the desired porosity, and synthesize the nanostructured material therein. Very suitable the polymer matrix may be imbibed with a solution of a metal salt which may then be precipitated the metal salt in the form of a metal hydroxide or metal oxide in the matrix.

### The polymer matrix in the electrosorption filter

A porous polymer matrix is used in order to immobilize the nanostructured sorption material and to prevent unwanted leakage of nanomaterials. The porous structure of the polymer matrix will allow for the trapping of molecules of which the removal from the fluid is sought such as the ionic solutes, small and middle molecules such as urea, creatinine and billirubin.

In other to provide for a matrix having a specific pore size, the polymer in preferred embodiments is a cross-linked polymer. The porosity of the polymer matrix is crucial for enabling selectivity: a high diffusion rate towards the sorption sites is sought for the toxins to be removed, while specific blood components such as albumin need to be prevented for sorption. Since an albumin molecule is approximately an 80 Angstrom diameter sphere, a suitable pore size for the polymer matrix in applications for blood (plasma) or dialysate purification would be less than 80 Angstrom (less than 8 nm).

In a preferred embodiment the polymer with the nanostructured sorption materials can be electrically surface charged via electrodes. In another preferred embodiments the polymer is a chemically charged polymer.

Polymers used in aspects of the invention may be synthetic or natural polymers. Natural polymers (biopolymers) suitably comprise a cross-linked carbohydrate or protein, made of oligomeric and polymeric carbohydrates or proteins. The biopolymer is preferably a polysaccharide. Among these, the "starch family", including amylose, amylopectin and dextrins, is especially preferred. Other suitable polysaccharides include glucans and celllulose. A preferred cellulose is carboxymethylcellulose (CMC, e.g. AKUCELL from AKZO Nobel).

Carbohydrates which can thus be used are carbohydrates consisting only of C, H and O atoms. Preferably, oligomeric carbohydrates with a degree of polymerization (DP) from DP2 on or polymeric carbohydrates from DP50 on are used. Most preferably the oxidized starch is crosslinked. A preferred crosslinking agent is di-epoxide. Very suitably, the crosslinking level is between 0.1 and 25%, more preferably between 1 and 5%, and most preferably between 2.5 and 3.5%.

Proteins which can be used include albumin, ovalbumin, casein, myosin, actin, globulin, hemin, hemoglobin, myoglobin, gelatin and small peptides. In the case of proteins, proteins obtained from hydrolysates of vegetable or animal material can also be used. Particularly preferred protein polymers are gelatin or a derivative of gelatin.

Also suitable mixtures of carbohydrates (e.g. copolymers) or mixtures of proteins can be used.

In order to provide for a charged polymer, the carbohydrate polymer may for instance be modified by oxidation, substitution with cationic functional groups or with carboxymethyl groups, or by esterification with e.g. acetyl groups. Particularly preferred carbohydrate polymers are chosen from the group consisting of starch or a derivative of starch, cellulose or a derivative of cellulose, pectin or a derivative of pectin.

Modification of the polymers can be accomplished by oxidation, substitution with cationic functional groups or carbonyl and/or carboxymethyl groups and/or esterifying with e.g. acetyl groups. Although in the latter case no charge is added, it is used to make the polymer more hydrophobic to allow complexing of the polymer with nanostructured sorption materials that have little or no charge.

Generally the polymers will be modified before cross-linking and gelation. It is possible to modify the polymer after cross-linking and gelation only if cross-linking is performed by ether-formation. The person skilled in the art will know how to modify the polymers specified in the invention to provide them with the mentioned groups.

The charge of the cross-linked polymer can be negative or positive depending on the type of polymer, the type of modification and the type of cross-linking. Advantageously, the polymers are of considerable size, i.e. 30 kD or more. This allows for the ready formation of a gel upon cross-linking and it allows for the formation of a lattice, which is capable of taking up the nanostructured sorption material.

The selectivity of the sorption material of the present invention can further be enhanced by loading the material with specific molecule catchers or receptors receptors such as antibodies, prosthetic groups or carboxyl groups (general: binding partners). For selective binding of proteins or degenerated proteins, antibodies are very suitable, e.g. from the immunoglobulin superfamily (IgSF), prosthetic groups e.g. from lipid and vitamin derivatives or metal ion such as gold, iron, zinc, magnesium, calcium that covalently bond to proteins, as well as carboxyl groups that can bind to proteins by forming peptide bonds. Such selective absorbing agents can further enhance the selectivity of the filter pad for toxic proteins.

In a preferred embodiment, the device of the present invention comprises means for regulating the content of minerals and other substances in the blood plasma via selective sorption and controlled release.

In a preferred embodiment therefore, the device for the removal of toxic substances from blood from a patient, comprises a hemofilter and an electrosorption filter, wherein the electrosorption filter removes toxic solutes, small and middle-sized molecules from the blood based, and includes such functions as selective sorption, controlled release and antimicrobial control.

In a most preferred embodiment, the plasmafilter of hemofilter, and electrosorption filter are separate parts. For instance, the plasmafilter or hemofilter may consist of an existing, commercially available plasmafilter, albuflow filter or high flux hemofilter and the electrosorption filter may consist of a cartridge with built in electrodes. In this cartridge also the sorption material is contained. The sorption material can be held in a porous envelop to form a filter pad.

The device of the present invention can be used for filtering or purification of blood of patients with a (developing) renal failure. In a preferred embodiment, the device takes the form of a wearable artificial kidney device, but can also be embodied in desktop sized equipment or in adapted hemodialysis or peritoneal dialysis equipment.

The artificial kidney is able to perform some of the functions which normally will be done by a properly functioning human or animal kidney, in particular filtering of blood and regulation and control of the content of substances in the blood.

### Experimental results

### Example 1: Purification of ultrafiltrate

A prototype has been built as depicted in figures 3 and 4 This setup has been tested in a dynamic test using blood plasma and a high flux filter to produce ultrafiltrate. The electrosorption and decomposition unit was filled with 25 grams of a nanoclay/polymer composite and 10 grams of a nanoporous active carbon. Ti/RuO2/TiO2 catalytic electrodes were used for the decomposition of urea at a voltage of 3 V per electrode pair (mild electro-oxidation) and 5 V per electrode pair (strong electrod-oxidation). The total electrode surface amounted to 600 cm². The ultrafiltrate flow was set to 50 ml/min. Concentrations of toxins before and after the sorption and decomposition unit were measured. After two hours the following results,being representative for the performance of the device, have been found.

**Table: Purification of an ultrafiltrate flow (50 ml/min) with an electrosorption/decomposition unit filled with 35 g sorbents**

| Concentration ratio outlet/inlet sorbent unit | only oxidation mild (3V) | only oxidation strong (5V) | only sorbents | mild oxidation + sorbents |
|---|---|---|---|---|
| potassium | 1 | 1 | 0,85 | 0,8 |
| phosphate | 1 | 1 | 0,75 | 0,75 |
| creatinine | 0,97 | 0,84 | 0,66 | 0,37 |
| Urea | 0,91 | 0,75 | 0,8 | 0,43 |
| b2m | 0,52 | 0,06 | 0,01 | 0 |
| glycine (SIR) | 0,94 | 0,23 | 0,7 | 0,02 |
| tryptophane | 0,16 | 0,01 | 0,01 | 0,03 |
| glutamin acid | 0,36 | 0,01 | 0,46 | 0,01 |

### Example 2: Purification of dialysate

A prototype has been built as depicted in figures 3 and 4 This setup has been tested in a dynamic test using dialysate from a dialysis center. The toxin concentrations in the dialysate are considerably lower (about a factor 2) than in the ultrafiltrate from example 1. The efficiency of the sorption and decomposition proces is therefore somewhat reduced. The electrosorption and decomposition unit was filled with 35 grams of a zeolite (4A) and 15 grams of a metaloxyhydroxide sorbent (FeOOH). Ti/RuO2/TiO2 catalytic electrodes were used for the decomposition of urea at a voltage of 3,5 V per electrode pair (mild electro-oxidation). The total electrode surface amounted to 600 cm². The dialysate flow was set to 50 ml/min. Concentrations of toxins before and after the sorption and decomposition unit were measured. After one hour the following results, being representative for the performance of the device, have been found.

**Table: Purification of a dialysate flow (50 ml/min) with the electrosorption/decomposition unit filled with 50 g sorbents**

| Concentration ratio outlet/inlet sorbent unit | mild oxidation (3,5 V) + sorbents |
|---|---|
| potassium | 0,84 |
| phosphate | 0,84 |
| creatinine | 0,92 |
| Urea | 0,86 |

## Claims

1. A device for the removal of substances from biofluid in particular toxic substances from dialysate fluid, blood, blood plasma or ultra filtrate, **characterized in that** said device comprising:
i) a housing having an inlet for entry of dialysate fluid, blood, blood plasma or ultrafiltrate into said housing, an outlet for the removal of purified dialysate fluid, blood, blood plasma or ultrafiltrate and excess fluid from said housing, and a conduit connecting said inlet with said outlet
ii) an electrocatalytic decomposition filter (8) for removing toxins, toxic solutes, toxic small and middle-sized molecules and protein bound toxins from the dialysate fluid, blood, bloodplasma or ultrafiltrate, said electrocatalytic decomposition filter being contained in said conduit such that said dialysate fluid, blood, blood plasma or ultrafiltrate must pass through said electrocatalytic decomposition filter when flowing from said inlet to said outlet and said electrocatalytic decomposition filter comprising:
a) a set of electrodes (7) with an electro-catalytic surface for decomposition and gasification of toxins via electro-oxidation
b) or a set of electrodes (7) that are in direct electrical contact with porous materials that have been coated with elecro-catalytic material
c) power source to provide the electrodes with an electrical charge in order to activate the electrocatalytic electrode surface
d) an electronic means to control and switch the electrical charge on the electrodes in view of resorting and release of toxins and to prevent built-up of deposits on the electrodes.
iii) an electrosorption filter (10) for removing toxins, toxic solutes, toxic small and middle-sized molecules and protein bound toxins from the dialysate fluid, blood, bloodplasma or ultrafiltrate, said electrosorption filter being contained in said conduit such that said dialysate fluid, blood, blood plasma or ultrafiltrate must pass through said electrosorption filter when flowing from said inlet to said outlet and said electrosorption filter comprising:
a) a sorption material selected from the group consisting of:
1) a nanostructured sorption material;
2) a porous polymer matrix, wherein the pores in said matrix are of a size that allows the entry into said matrix of toxic substances sought to be removed from said liquid and/or preventing the entry into said matrix of substances not sought to be removed from said liquid; and
3) a nanostructured sorption material captured in a porous polymer matrix, wherein the pores in said porous polymer matrix are of a size that allows the entry into said matrix of substances sought to be removed from said liquid while preventing the escape of said nanostructured sorption material and/or preventing the entry into said matrix of substances not sought to be removed from said ,liquid; said sorption filter being contained in a housing;
b) a set of electrodes (7) coated with or in electrical contact with the sorption material;
c) a power source to provide the electrodes with an electrical charge and generate an electrical field strength in the sorption medium.

2. Device according to claim 1, **characterized in that** the electrodes for the electrocatalytic filter are planar, circular, tubular or thread electrodes made from:
i) metals such as Pt, Ni, Ti, Ir, Sn, Ta and Ru,
ii) oxides such as Ti02, Ru02, Ri02, Sn02, Ta2O3, NiO
iii) oxyhydroxides such as TiO(OH)2, RuO(OH)2, RiO(OH)2, SnO(OH)2, NiOOH, TaOOH
iv) hydroxides such as: Ti(OH)4, Ru(OH)4, Ri(OH)4, Sn(OH)4, Ni(OH)2, Ta(OH)3

3. Device according to claim 1 or 2, **characterized in that** the electro sorption filter and electrocatalytic decomposition filter are combined into one filter compartment wherein the sorption material is contained in between the electrocatalytic electrodes

4. Device according to claim 1, 2 or 3, **characterized in that** the nanostructured sorption material is selected from the group consisting of:
i) nanoparticles or nanocrystalline materials, preferably metal silicates such as nanoclays, preferably an exfoliated nanoclay, metal hydroxides, preferably layered double hydroxides like a nano-hydrotalcite or pure metaloxide nanoparticles;
ii) nanoporous materials, preferable selected from zeolites, mesoporous systems, carbonaceous nanomaterials and metal organic frameworks;
iii) nanocomposites;
iv) nanofibers, and
v) any combination of the above.

5. Device according to claim 1, 2, 3 or 4, **characterized in that** said nanostructured sorption material captured in a porous polymer matrix is based on a cross-linked polymer and/or a charged polymer, preferably a biopolymer selected from selected from carbohydrates and proteins, wherein said carbohydrate is selected form an oxidized crosslinked starch and a carboxymethyl cellulose.

6. Device according to claim 5, **characterized in that** the pores in said matrix are of a size that prevents the entry into said matrix of albumin.

7. Device according to any one of the preceding claims, **characterized in that** said electrocatalytic decomposition and electrosorption filter is provided in the form of a cartridge with said electrodes coated with said sorption material or with said electrodes surrounded by a porous envelop containing said sorption material.

8. Device according to any one of the preceding claims, **characterized in that** said electrocatalytic decomposition and electrosorption filter is combined with a permeable envelop to form a filter pad comprising an envelop surrounding a filter pad contents, wherein the envelop of said pad comprises a permeable membrane and the contents of said pad comprise said sorption material.

9. Device according to any one of the preceding claims, **characterized in that** said device further comprises a plasmafilter for separating patient blood plasma from patient blood cells.

10. Device according to any one of the preceding claims, **characterized in that** said device further comprises a hemofilter for separating patient ultrafiltrate from patient blood cells or to allow blood-dialysate toxin exchange in a dialysis setup.

11. Device according to any of the preceding claims, **characterized in that** said device further comprises an electrosorption filter for removing toxins, small and middle-sized molecules from the patient blood plasma.

12. Device according to claims 8 and 10, **characterized in that** said plasmafilter and said electrosorption filter are combined to form the filter pad wherein said permeable membrane is formed by said plasmafilter.

13. Device according to claims 9 and 10, **characterized in that** said hemofilter and said electrosorption filter are combined to form the filter pad wherein said permeable membrane is formed by said hemofilter.

14. Device according to any one of the preceding claims, **characterized in that** said device further comprises means for supplementing said dialysate fluid and/or said, (purified) blood plasma with at least one substance selected from the group consisting of vitamins, minerals, anticoagulants, anti microbial agents and other medicaments.

15. Device according to any one of the preceding claims, **characterized in that** said device further comprises ion exchange system.

## Patentansprüche

1. Einrichtung zum Entfernen von Stoffen aus Bioflüssigkeit, insbesondere toxische Stoffe aus Dialysierflüssigkeit, Blut oder Blutplasma, **dadurch gekennzeichnet, dass** die genannte Einrichtung umfasst:
i) ein Gehäuse (3) mit einem Einlass (5) für den Zufluss von Dialysierflüssigkeit, Blut oder Blutplasma in das genannte Gehäuse, einem Auslass (6) zum Entfernen der gereinigten Dialysierflüssigkeit, des Blutes oder Blutplasmas und der überschüssigen Flüssigkeit aus dem genannten Gehäuse, und einem Kanal (7), der den genannten Einlass mit dem genannten Auslass verbindet;
ii) eine elektrokatalytische Zersetzung Filter zur Entfernung von Toxinen, toxische gelöste Stoffe, giftig kleinen und mittelgroßen Moleküle und Protein gebunden Giftstoffe aus dem Dialysierflüssigkeit, Blut, Blutplasma oder Ultrafiltrat, die elektrokatalytische Zersetzung Filters in der Leitung enthalten, so dass das Dialysefluid, Blut, Blutplasma oder Ultrafiltrat muß durch die elektrokatalytische Zersetzung Filter beim Fließen passieren dem Einlass zu dem Auslass und dem elektrokatalytische Zersetzung Filter aufweist:
a) eine Gruppe von Elektroden mit einem elektrokatalytischen Oberfläche zur Zersetzung und Vergasung von Toxinen über die Elektrooxidation
b) oder eine Reihe von Elektroden, die in direktem elektrischen Kontakt mit porösen Materialien, die mit elekrokatalytischen Material beschichtet worden sind,
c) Stromquelle, die Elektroden mit einer elektrischen Ladung, um die elektrokatalytische Elektrode Oberfläche zu aktivieren bereitzustellen
d) eine elektronische Einrichtung zur Steuerung und schalten Sie die elektrische Ladung auf den Elektroden angesichts der resorbierenden und Freisetzung von Toxinen und gebaut von Ablagerungen auf den Elektroden zu verhindern;
iii) ein Elektrosorptionsfilter (11, 37) zum Entfernen von Toxinen, gelösten toxischen Stoffen, kleinen und mittelgroßen toxischen Molekülen und eiweißgebundenen Toxinen aus der Dialysierflüssigkeit, dem Blut oder Blutplasma, wobei sich das genannte Elektrosorptionsfilter in dem genannten Kanal befindet, sodass die genannte Dialysierflüssigkeit, das Blut oder das Blutplasma beim Weg vom genannten Einlass zum genannten Auslass durch das genannte Elektrosorptionsfilter strömen muss und wobei das genannte Elektrosorptionsfilter Folgendes umfasst:
a) einem Sorptionsmaterial aus der Gruppe gewählt, bestehend aus:
1) einem nanostrukturierten Sorptionsmaterial;
2) einer porösen Polymermatrix, wobei die Poren in der genannten Matrix eine derartige Größe haben, dass die Zufuhr der aus der Flüssigkeit zu entfernenden toxischen Stoffe zur genannten Matrix ermöglicht wird und/oder die Zufuhr von Stoffen zur genannten Matrix, die nicht aus der genannten Flüssigkeit entfernt werden sollen, verhindert wird; und
3) einem in einer porösen Polymermatrix eingeschlossenen, nanostrukturierten Sorptionsmaterial, wobei die Poren in der genannten porösen Polymermatrix eine derartige Größe haben, dass die Zufuhr der aus der Flüssigkeit zu entfernenden Stoffe zur genannten Matrix ermöglicht wird, während ein Entweichen des genannten nanostrukturierten Sorptionsmaterials und/oder die Zufuhr von Stoffen zur genannten Matrix, die nicht aus der genannten Flüssigkeit entfernt werden sollen, verhindert wird; das Sorptionsfilter befindet sich dabei in einem Gehäuse;
b) einem Satz Elektroden (13, 15), verkleidet mit oder in elektrischem Kontakt mit dem Sorptionsmaterial;
c) einer Stromquelle zur Versorgung der Elektroden mit einer elektrischen Ladung, um eine elektrische Feldstärke im Sorptionsmedium und um Wasserstoffionen H+- und Hydroxyl OH--Ionen zur Regeneration zu erzeugen.

2. Vorrichtung nach Anspruch 1, **dadurch gekennzeichnet, dass** die Elektroden zur elektrokatalytischen Filters sind planar, kreisförmig, röhrenförmig oder Fadenelektroden hergestellt aus:
i) Metallen wie Pt, Ni, Ti, Ir, Sn, Ta und Ru,
ii) Oxiden wie TiO 2, RuO 2, Rio2, SnO2, Ta2O3, NiO
iii) Oxyhydroxiden wie TiO (OH) 2, RuO (OH) 2, Rio (OH) 2, SnO (OH) 2, NiOOH, TaOOH
iv) Hydroxide, wie: Ti (OH) 4, Ru (OH) 4, Ri (OH) 4, Sn (OH) 4, Ni (OH) 2, Ta (OH) 3

3. Vorrichtung nach Anspruch 1 oder 2, **dadurch gekennzeichnet, dass** der Elektrosorption Filter und elektrokatalytische Zersetzung Filter werden in einer Filterkammer, wobei das Sorptionsmaterial in zwischen den elektrokatalytischen Elektroden enthaltenen kombinierten.

4. Einrichtung nach Anspruch 1, 2 oder 3, **dadurch gekennzeichnet, dass** das nanostrukturierte Sorptionsmaterial aus einer Gruppe gewählt wurde, bestehend aus:
i) Nanopartikeln oder nanokristallinem Material, vorzugsweise Metallsilikate wie Nanoton, bevorzugt expandierter Nanoton, Metallhydroxide, bevorzugt geschichtete Doppelhydroxide wie Nanohydrotalcit oder reine Metaloxidnanopartikel;
ii) nanoporösem Material, vorzugsweise aus Zeolithen gewählt, mesoporösen Systemen, kohlenstoffhaltigem Nanomaterial und metallorganischen Strukturen;
iii) Nanocompositen;
iv) Nanofasern; und
v) jeder Kombination des Obigen.

5. Einrichtung nach Anspruch 1, 2, 3 oder 4, **dadurch gekennzeichnet, dass** das nanostrukturierte Sorptionsmaterial in einer porösen Polymermatrix eingeschlossen ist, basierend auf einem vernetzten Polymer und/oder einem geladenen Polymer, vorzugsweise ein Biopolymeer, gewählt aus den Kohlenhydraten und Eiweißen, wobei das genannte Kohlenhydrat aus einer oxidiert vernetzten Stärke und Carboxymethylcellulose gewählt ist.

6. Einrichtung nach Anspruch 5, **dadurch gekennzeichnet, dass** die Poren in der genannten Matrix eine derartige Größe haben, dass die Zufuhr von Albumin zur genannten Matrix verhindert wird.

7. Einrichtung nach einem der obigen Ansprüche, **dadurch gekennzeichnet, dass** das Elektrosorptionsfilter die Form einer Kassette hat, wobei die genannten Elektroden mit Sorptionsmaterial verkleidet sind, oder wobei die genannten Elektroden von einer porösen Umhüllung, in der sich das Sorptionsmaterial befindet, umgeben sind.

8. Einrichtung nach einem der obigen Ansprüche, **dadurch gekennzeichnet, dass** das genannte Elektrosorptionsfilter mit einer durchlässigen Umhüllung kombiniert ist, um ein Filterkissen zu bilden, dass eine Umhüllung hat, die einen Filterkisseninhalt umgibt, wobei die Umhüllung des genannten Kissens eine durchlässige Membrane enthält und der Inhalt des genannten Kissens das genannte Sorptionsmaterial umfasst.

9. Einrichtung nach einem der obigen Ansprüche, **dadurch gekennzeichnet, dass** die Einrichtung ferner einen Hämofilter (9, 31) zum Scheiden von Blutplasma von Blutzellen des Patienten enthält.

10. Vorrichtung nach einem der vorhergehenden Ansprüche, dass die Vorrichtung weiterhin aufweist einen Hämofilter zur Trennung Patienten Ultrafiltrat aus Patientenblutzellen oder Blut Dialysat Toxin Austausch in einer Dialyseeinrichtung zu gestatten.

11. Vorrichtung nach einem der vorhergehenden Ansprüche, dass die Vorrichtung weiter aufweist eine Elektrosorption Filter zum Entfernen von Toxinen, kleine und mittlere Moleküle aus dem Patientenblutplasma.

12. Vorrichtung nach den Ansprüchen 8 und 10, daß der Plasmafilter und die genannten Elektrosorption Filter werden kombiniert, um die Filter-Unterlage zu bilden, wobei die durchlässige Membran durch das Plasmafilter gebildet.

13. Einrichtung nach Anspruch 9 und 10, **dadurch gekennzeichnet, dass** das genannte Hämofilter und das genannte Elektrosorptionsfilter zur Bildung des Filterkissens kombiniert sind, wobei die genannte durchlässige Membrane vom genannten Hämofilter gebildet wird.

14. Einrichtung nach einem der obigen Ansprüche, **dadurch gekennzeichnet, dass** die Einrichtung ferner Mittel zum Nachfüllen der genannten Dialysierflüssigkeit und/oder des (gereinigten) Blutplasmas enthält mit mindestens einem Stoff, gewählt aus der Gruppe, bestehend aus Vitaminen, Mineralien, Antikoagulantien, antimikrobiellen Mitteln und anderen Medikamenten.

15. Vorrichtung, nach einem der vorhergehenden Ansprüche, dass die Vorrichtung weiterhin aufweist Ionenaustauschsystem.

## Revendications

1. Dispositif pour l'élimination de substances présentes dans du bioliquide, notamment de substances toxiques présentes dans du liquide de dialyse, du sang ou du plasma sanguin, **caractérisé en ce que** le dispositif précité comprend:
i) un boîtier (3) ayant une admission (5) pour l'apport de liquide de dialyse, de sang ou de plasma sanguin dans le boîtier précité, un orifice d'écoulement (6) pour extraire du boîtier précité le liquide de dialyse, le sang ou le plasma sanguin et le liquide en excès purifiés, et un conduit (7) reliant l'admission précitée avec l'orifice d'écoulement précité.
ii) un filtre de décomposition électrocatalytique pour l'élimination des toxines, des solutés toxiques, toxiques petites et moyennes molécules et des protéines liées les toxines du fluide de dialysat, sang, plasma sanguin ou ultrafiltrat, ledit filtre de décomposition électrocatalytique étant contenu dans ledit conduit de telle sorte que ledit fluide de dialysat, sang, le plasma sanguin ou ultrafiltrat doit passer à travers ledit filtre de décomposition électrocatalytique lorsque écoulement depuis ladite entrée vers ladite sortie et ledit filtre de décomposition électrocatalytique comprenant:
a) un jeu d'électrodes avec une surface électro-catalytique pour la décomposition et de la gazéification des toxines par électro-oxydation
b) ou un ensemble d'électrodes qui sont en contact électrique direct avec des matières poreuses qui ont été revêtues d'un matériau catalytique elecro
c) source d'alimentation pour fournir les électrodes avec une charge électrique afin d'activer la surface de l'électrode électrocatalytique
d) un moyen électronique de contrôle et de commande de la charge électrique sur les électrodes en vue de la résorption et la libération de toxines et prévenir intégré de dépôts sur les électrodes;
iii) un filtre d'électrosorption (11, 37) pour éliminer du liquide de dialyse, du sang ou du plasma sanguin les toxines, les substances toxiques dissoutes, les petites et moyennes molécules toxiques et les toxines liées aux protéines, où le filtre d'électrosorption précité est contenu dans le conduit précité de telle sorte que le liquide de dialyse, le sang ou le plasma sanguin précité est obligé de passer à travers le filtre d'électrosorption précité lorsqu'il s'écoule depuis l'admission précitée vers l'orifice d'écoulement précité, et où le filtre d'électrosorption précité comprend:
a) un matériau de sorption choisi parmi le groupe comprenant:
1) un matériau de sorption nanostructuré;
2) une matrice polymère poreuse, où les pores se trouvant dans la matrice précitée ont une grosseur permettant l'entrée dans la matrice précitée des substances toxiques que l'on cherche à éliminer du liquide précité et/ou empêchant l'entrée dans la matrice précitée des substances que l'on ne cherche pas à éliminer du liquide précité; et
3) un matériau de sorption nanostructuré emprisonné dans une matrice polymère poreuse, où les pores se trouvant dans la matrice polymère poreuse précitée ont une grosseur permettant l'entrée dans la matrice précitée des substances que l'on cherche à éliminer du liquide précité, tout en empêchant le matériau de sorption nanostructuré précité de s'échapper et/ou en empêchant l'entrée dans la matrice précitée des substances que l'on ne cherche pas à éliminer du liquide précité; où le filtre de sorption précité est contenu dans un boîtier;
b) une paire d'électrodes (13, 15) revêtues du ou en contact électrique avec le matériau de sorption;
c) un bloc d'alimentation pour apporter une charge électrique aux électrodes afin de générer une intensité de champ électrique dans le milieu de sorption et afin de générer les ions hydrogène H+ et hydroxyde OH- pour la régénération.

2. Dispositif selon la revendication 1, **caractérisé en ce que** les électrodes du filtre électrocatalytique des électrodes planes, circulaires, tubulaires ou fils fabriqués à partir de:
i) des métaux tels que Pt, Ni, Ti, Ir, Sn, Ta et Ru,
ii) les oxydes tels que TiO2, RuO2, RIO2, SnO2, Ta2O3, NiO
iii) tels que des oxyhydroxydes de TiO (OH) 2, RuO (OH) 2, Rio (OH) 2, SnO (OH) 2, NiOOH, TaOOH
iv) des hydroxydes tels que: Ti (OH) 4, Ru (OH) 4, Ri (OH) 4, Sn (OH) 4, Ni (OH) 2, Ta (OH) 3

3. Dispositif selon la revendication 1 ou 2, **caractérisé en ce que** le filtre et le filtre à électrosorption décomposition électrocatalytique sont combinés en un seul bloc de filtration dans lequel la matière de sorption est contenu dans entre les électrodes électrocatalytiques.

4. Dispositif selon la revendication 1, 2 ou 3, **caractérisé en ce que** le matériau de sorption nanostructuré est choisi parmi le groupe comprenant:
i) des nanoparticules ou matériaux nanocristallins, de préférence des silicates métalliques tels que les nanoargiles, de préférence une nanoargile exfoliée, des hydroxydes métalliques, de préférence des hydroxydes doubles stratifiés comme le nanohydrotalcite, ou des nanoparticules d'oxydes métalliques pures;
ii) des matériaux nanoporeux, de préférence choisis parmi les zéolithes, les systèmes mésoporeux, les nanomatériaux carbonés et les réseaux métal-organiques;
iii) des nanocomposites;
iv) des nanofibres, et
v) toute combinaison de ce qui est décrit ci-dessus.

5. Dispositif selon la revendication 1, 2, 3 ou 4, **caractérisé en ce que** le matériau de sorption nanostructuré précité emprisonné dans une matrice polymère poreuse est à base de polymère réticulé et/ou de polymère chargé, de préférence un biopolymère choisi parmi les glucides et les protéines, où le glucide précité est choisi parmi un amidon réticulé oxydé et une carboxyméthylcellulose sodique.

6. Dispositif selon la revendication 5, **caractérisé en ce que** les pores se trouvant dans la matrice précitée ont une grosseur empêchant l'entrée de l'albumine dans la matrice précitée.

7. Dispositif selon une des revendications précédentes, **caractérisé en ce que** le filtre d'électrosorption précité est fourni sous la forme d'une cartouche comprenant les électrodes précitées revêtues du matériau de sorption précité ou comprenant les électrodes précitées entourées d'une gaine poreuse contenant le matériau de sorption précité.

8. Dispositif selon une des revendications précédentes, **caractérisé en ce que** le filtre d'électrosorption précité est combiné avec une gaine perméable de façon à former un coussinet de filtre comprenant une gaine qui entoure un contenu de coussinet de filtre, où la gaine du coussinet précité comprend une membrane perméable, et le contenu du coussinet précité comprend le matériau de sorption précité.

9. Dispositif selon une des revendications précédentes, **caractérisé en ce que** le dispositif précité comprend de plus un hémofiltre (9, 31) pour séparer le plasma sanguin du patient des cellules sanguines du patient.

10. Dispositif selon l'une quelconque des revendications précédentes, **caractérisé en ce que** ledit dispositif comprend en outre un hémofiltre pour séparer l'ultrafiltrat du patient à partir de cellules de sang de patients ou pour permettre au sang-dialysat échange de la toxine dans une installation de dialyse.

11. Dispositif selon l'une quelconque des revendications précédentes, **caractérisé en ce que** ledit dispositif comprend en outre un filtre pour éliminer les toxines électrosorption, les petites et moyennes molécules du plasma sanguin du patient.

12. Dispositif selon les revendications 8 et 10, **caractérisé en ce que** ledit et ledit plasmafiltre électrosorption filtre sont combinés pour former le tampon de filtre, dans lequel ladite membrane perméable est formée par ledit plasmafiltre.

13. Dispositif selon la revendication 9 et 10, **caractérisé en ce que** l'hémofiltre précité et le filtre d'électrosorption précité sont combinés de façon à former le coussinet de filtre, où la membrane perméable précitée est formée par l'hémofiltre précité.

14. Dispositif selon une des revendications précédentes, **caractérisé en ce que** le dispositif précité comprend de plus des moyens pour compléter le liquide de dialyse précité et/ou le plasma sanguin (purifié) avec au moins une substance prise parmi le groupe comprenant des vitamines, des minéraux, des anticoagulants, des agents antimicrobiens et d'autres médicaments.

15. Dispositif selon l'une quelconque des revendications précédentes, **caractérisé en ce que** ledit dispositif comprend en outre le système d'échange d'ions.
